# EUROPEAN PATENT APPLICATION

(11) **EP 4 531 023 A1**
(43) Date of publication of application: **02.04.2025**
(21) Application number: 23862090.0
(22) Date of filing: 31.07.2023
(51) Int. Cl.: G09B 9/00, G09B 23/28, G09B 5/06, A61N 1/39

(54) **INTELLIGENT CARDIOPULMONARY RESUSCITATION TRAINING AND ASSESSMENT SYSTEM AND TRAINING AND ASSESSMENT METHOD**

(30) Priority: 09.09.2022 CN 202211101881; 09.09.2022 CN 202211101875
(71) Applicant: Qingdao Bright Medical Manufacturing Co., Ltd., Qingdao, Shandong 266107 (CN)
(72) Inventor: ZHAO, Dezheng, Qingdao, Shandong 266107 (CN)
(74) Representative: Cabinet Beau de Loménie
(86) International application number: PCT/CN2023/110130
(87) International publication number: WO 2024/051390

(57) **Abstract**

The present invention provides an intelligent cardiopulmonary resuscitation (CPR) training and assessment system and a training and assessment method. The system comprises: a cabinet housing, and a human body sensor, a camera, an identity card reader, a switching power supply, a touch display screen and a control system on the cabinet housing. A CPR simulator and an AED simulator are arranged on the inner side surface of a lower flap of the lower part of the cabinet housing, and are characterized in that: a CPR simulator control circuit board arranged in the CPR simulator is connected to the control system, and the control system comprises a CPR training module, a CPR assessment module, an AED training module, an AED assessment module, a touch screen driving module and a video playback module. The training and assessment method comprises a CPR training method, a CPR assessment method, an AED training method and an AED assessment method. The system has a simple and reasonable structure and many functions, and is convenient to operate and use; the CPR training and assessment method has detailed and highly operable methods and steps, so that students can quickly master an operation and use method.

## Description

### Technical Field

The present invention belongs to the field of medical emergency equipment technology, primarily involving improvements in automatic external defibrillators (AEDs) and CPR emergency training and assessment devices. Specifically, it pertains to a smart CPR training and assessment system and method.

### Background

Emergency medical services are a critical part of healthcare. While automated external defibrillators (AEDs) have been widely installed in public spaces nationwide, many people still don't know how to use them effectively or perform cardiopulmonary resuscitation (CPR).

There is a significant shortage of individuals trained in emergency medical response, making it challenging to meet society's' emergency needs. Therefore, it is crucial and urgent to boost public awareness, experiential learning, and training in locations where AEDs are available.

Existing CPR training and assessment systems have limitations in terms of functionality, convenience, and safety. They often do not provide a user-friendly, safe, and reliable experience for trainees. Moreover, these systems tend to be structurally complex, expensive, and poorly designed, making maintenance difficult and hindering their widespread adoption.

Chinese Patent (Application No.: 201710392317.8) describes an AED training and assessment device and method, which includes an AED server, central controller, housing, display screen, and AED teaching apparatus. The central controller features modules for AED teaching, sensor signal processing, and touch screen driving, and is connected to the AED server. Additionally, it includes modules for AED training and assessment, identity recognition and verification, and certificate printing. The housing is equipped with an identity card scanning and recognition device, printer, and camera, all connected to the central controller. This setup allows the public to easily learn and use AEDs on the training and assessment device, enabling on-site assessment and certification for qualified trainees. This promotes the use of defibrillation technology and ensures that AEDs in public spaces can save lives.

However, the above patent application has the following issues:
The training and assessment system lacks comprehensive and functional modules.

The CPR training and assessment methods, as well as the AED training and assessment methods, lack detailed and specific operational steps, making it difficult for trainees to quickly master the techniques.

Therefore, there is an urgent need to design and develop a smart CPR training and assessment system and method. This new system should have a simple and rational structure, multiple functions, and be easy to operate. The CPR training and assessment methods should be detailed and specific, enabling trainees to quickly master the techniques. This is a pressing technical issue in the field.

### Technical Problem

The present invention aims to address the aforementioned problems in existing technologies by providing a smart CPR training and assessment system and method. The system has a simple and rational structure, multiple functions, and is easy to operate. The CPR training and assessment methods are detailed and specific, enabling trainees to quickly master the techniques.

### Technical Solution

The objectives of the present invention are achieved through the following technical solutions:
A smart cardiopulmonary resuscitation (CPR) training and assessment system, comprising a cabinet body, CPR simulation manikin, AED simulation machine, switch power supply, touch display screen, and control system. A front panel of an upper part of the cabinet body is equipped with a human body sensor, camera, and ID card reader. The upper part of the cabinet body's two side walls each have a speaker. The AED simulation machine, touch display screen, speakers, human body sensor, camera, and ID card reader are all connected to the control system. The upper part of the cabinet body houses the touch display screen and control system. A cabinet door in front of a compartment of the lower part of the cabinet body opens downward. The CPR simulation manikin and AED simulation machine are mounted on the inner side of the downward-opening door. The CPR simulation manikin contains a CPR simulation manikin control circuit board, which is connected to the control system via a serial communication cable. The control system includes CPR training module, CPR assessment module, AED training module, AED assessment module, touch screen driver module, and video playback module.

Improvement to the above technical solution: The CPR simulation manikin includes a head shell and a torso shell. The torso shell contains a compression spring, with the lower end fixed to the inner back side of the torso shell. The upper end of the compression spring is sequentially fitted with a compression board and a chest board. Below the compression board is a compression depth sensor, which detects the depth of hand pressure applied by the user during CPR training or assessment. The bottom surface of the chest board has a compression position sensor, which detects the position of the user's hand pressure on the chest board during CPR training or assessment. The head shell has a breathing tube inserted into its mouth. The CPR simulation manikin control circuit board has an air pressure sensor, with the breathing tube connected to the air pressure sensor's air intake port to measure the blowing pressure during artificial respiration. The CPR simulation manikin control circuit board includes a central controller and a sensor signal processing module that is connected to the compression depth sensor, compression position sensor, and air pressure sensor.

Further improvement to the above technical solution: The CPR simulation manikin control circuit board is mounted on the inner back side of the torso shell. The compression depth sensor uses an ultrasonic probe, including an ultrasonic probe transmitter end mounted on the bottom surface of the compression board and an ultrasonic probe receiver end mounted on the CPR simulation manikin control circuit board. The compression position sensor includes a circuit board and several conductive silicone rubber blocks housed in an open-ended shell. The conductive silicone rubber blocks are embedded in the middle and peripheral parts of the bottom surface of the chest board. The open-ended shell is detachably mounted on the bottom surface of the chest board, covering all the conductive silicone rubber blocks. The circuit board is connected to the sensor signal processing module. The compression board is made of plastic, and the chest board is made of silicone, with the chest board covering the compression board. The chest board is covered with a chest skin.

Further improvement to the above technical solution: The AED simulation machine includes a box, a main unit inside the box, and two electrode pads connected to the main unit. The box has a cover, and the main unit has a button switch and a shock button. The box is mounted on the inner side of the downward-opening door near the head of the CPR simulation manikin. The chest board has two AED electrode pad position sensors to detect the position signals of the two electrode pads attached to the AED simulation machine. The CPR simulation manikin control circuit board includes a central controller and a sensor signal processing module that is connected to the AED electrode pad position sensors.

Further improvement to the above technical solution: The AED electrode pad position sensors include two Hall sensors mounted on the chest board and magnets mounted on the two electrode pads. The CPR simulation manikin control circuit board is mounted on the inner back side of the torso shell. The compression board is made of plastic, and the chest board is made of silicone, with the chest board covering the compression board. The chest board is covered with a chest skin.

Further improvement to the above technical solution: The torso shell of the CPR simulation manikin has a first aviation socket and a second aviation socket, both connected to the sensor signal processing module of the CPR simulation manikin control circuit board. An end of a signal line from the AED simulation machine's main unit is connected to a second aviation plug, which plugs into the second aviation socket. The first aviation plug from the control system plugs into the first aviation socket.

Further improvement to the above technical solution: The cabinet body is a vertical rectangular shell. The middle part of the cabinet body is an AED storage box with a door at the front. The AED storage box houses an AED. The top of the cabinet body has an alarm, which is connected to the switch power supply via a power cord. The door of the AED storage box has an electric lock connected to the control system, allowing the control system to control the electric lock to open the door and activate the alarm.

Further improvement to the above technical solution: At least one side edge of the inner side of the downward-opening door has a hinge connected to one end of a hydraulic rod. The other end of the hydraulic rod is hinged to the corresponding inner side of the compartment. The hydraulic rod uses a hydraulic damper. The downward-opening door or the cabinet body has an electric door lock connected to the control system via the CPR simulation manikin control circuit board, allowing the control system to control the electric door lock to automatically open the downward-opening door.

Further improvement to the above technical solution: The inner side of the downward-opening door near the torso shell of the CPR simulation manikin has a foam pad. The inner side wall of the compartment has a disinfectant bottle and a disposable CPR barrier disinfectant mask box.

Further improvement to the above technical solution: The touch display screen and control system are configured as a tablet computer. The top of the cabinet body has an antenna for the tablet computer to connect to a wireless local area network via WiFi. The control system also includes a wireless communication module connected to the antenna.

Further improvement to the above technical solution: The tablet computer includes a main screen and an additional screen. The main screen and the tablet computer's main unit are integrated and housed in a compartment in the upper part of the cabinet body. The additional screen is mounted on the inner side of the downward-opening door and is detachably connected to the downward-opening door. The additional screen is connected to the tablet computer's main unit.

The training and assessment method for the smart CPR training and assessment system includes CPR training methods and CPR assessment methods. The CPR training method comprises the following steps:
Step 1: After powering on, the touch display screen shows the main page. The user clicks on "CPR Training" on the main page, and the system navigates to the "CPR Training" page. The video window on the touch display screen starts playing video segment CPR I . The speaker begins playing the instructional audio: "CPR training requires you to perform chest compressions and artificial respiration on the simulation manikin. Are you ready? If you are ready, please click 'Start Training'".
Step 2: After the user clicks "Start Training," the video window starts playing video segment CPR II. The speaker begins playing the instructional audio: "The electric door lock has been opened. Please slowly pull open the cabinet door and lay it flat on the ground. Now, please kneel on the foam pad on the cabinet door with both knees, place your hands crossed on the midpoint of the line connecting the two nipples on the manikin's chest, and begin 30 chest compressions. The compression depth should be 5 to 6 centimeters. Please follow the rhythm of the sound" This audio is played at least 2 times.
Step 3: The user begins the chest compression action on the CPR simulation manikin. Once the system detects that the user has started the chest compression action, the video window starts playing video segment CPRIII, which demonstrates the correct chest compression technique. The speaker begins playing a rhythmic sound at a frequency of 100-120 beats per minute. The system monitors the user's compression action and provides real-time audio feedback through the speaker to correct the user's action: "Compression too deep," "Compression too shallow," "Insufficient recoil," or "Incorrect compression position".
Step 4: After the user completes 30 correct chest compressions, the video window starts playing video segment CPRIV. The speaker begins playing the instructional audio: "30 chest compressions completed. Please take a disinfectant mask from the disinfectant mask box, open the small package, place the mask on the CPR simulation manikin's mouth, and perform two artificial respirations".
Step 5: The user begins the artificial respiration action on the CPR simulation manikin. Once the system detects that the user has started the artificial respiration action, the video window starts playing video segment CPRV, which demonstrates the correct artificial respiration technique. The system monitors the user's artificial respiration action in real-time and provides real-time audio reminders through the speaker: "Correct breathing," "Breathing too strong," or "Breathing too weak".
Step 6: After the user completes 2 correct artificial respirations, the video window starts playing video segment CPRIII, which demonstrates the correct chest compression technique. The speaker begins playing the instructional audio: "Two artificial respirations completed. Please continue with 30 chest compressions".
Step 7: Repeat steps 3, 4, 5, and 6 until the user clicks "Exit Training" The video window starts playing the "Close Cabinet Door" video segment. The speaker begins playing the instructional audio: "Please slowly lift the cabinet door and close it firmly" When the "Close Cabinet Door" video segment ends, the system returns to the main page.

Improvement to the Above Technical Solution: The CPR assessment method includes the following steps:
Step 1: The user clicks on "CPR Assessment" on the main page of the touch display screen, and the system enters the identity recognition step. A dialog box appears: "Please place your ID card in the ID card scanning area". The speaker begins playing the instructional audio: "Please place your ID card in the ID card scanning area". If the ID card reader does not recognize a valid ID card within 30 seconds, the dialog box closes, and the system returns to the "Main Page".
Step 2: After the user successfully scans their ID card, the system navigates to the "CPR Assessment" page. The speaker begins playing the instructional audio: "CPR assessment requires you to perform chest compressions and artificial respiration on the simulation manikin. Are you ready? If you are ready, please click 'Start Assessment'".
Step 3: After the user clicks "Start Assessment," the speaker begins playing the instructional audio: "The electric door lock has been opened. Please slowly pull open the cabinet door and lay it flat on the ground. Now, please kneel on the foam pad on the cabinet door with both knees and begin 30 chest compressions".
Step 4: The user begins the chest compression action. Once the system detects that the user has started the chest compression action, the speaker plays a rhythmic sound at a frequency of 100-120 beats per minute.
Step 5: After the user completes 30 chest compressions, the speaker begins playing the instructional audio: "30 chest compressions completed. Please take a disinfectant mask from the disinfectant mask box, open the small package, place the mask on the CPR simulation manikin's mouth, and perform two artificial respirations".
Step 6: The user begins the artificial respiration action on the CPR simulation manikin.
Step 7: After the user completes 2 artificial respirations, the speaker begins playing the instructional audio: "Two artificial respirations completed. Assessment successful. Detailed scores are shown on the screen. CPR assessment ended. Now, please click 'Exit Assessment'" or "Two artificial respirations completed. Assessment failed. Detailed scores are shown on the screen. CPR assessment ended. Now, please click 'Exit Assessment'" .
Step 8: After the user clicks "Exit Assessment," the speaker begins playing the instructional audio: "Please slowly lift the cabinet door and close it firmly" The system returns to the main page.

Further Improvement to the Above Technical Solution: The AED training and assessment method includes the AED training method and the AED assessment method. The AED training method comprises the following steps:
Step 1: After powering on, the touch display screen shows the main page. The user clicks on "AED Training" on the main page, and the system navigates to the "AED Training" page. The video window on the touch display screen starts playing video segment AED I . The speaker begins playing the instructional audio: "AED training requires you to use the AED simulation machine to perform simulated defibrillation on the CPR simulation manikin. Are you ready? If you are ready, please click 'Start Training'" .
Step 2: After the user clicks "Start Training," the video window starts playing video segment AED II. The speaker begins playing the instructional audio: "The electric door lock has been opened. Please slowly pull open the cabinet door and lay it flat on the ground. Now, please kneel on the foam pad on the cabinet door and gently press the cover of the AED simulation machine to open it".
Step 3: After the user opens the cover of the AED simulation machine, the "AED Training" page displays "Cover Opened Correctly" The video window starts playing video segment AEDIII. The speaker begins playing the instructional audio: "The cover of the AED simulation machine has been correctly opened. Now, please take out the two electrode pads, peel off the adhesive film, and correctly attach them to the manikin's skin as indicated on the electrode pads".
Step 4: After the user attaches the two electrode pads, the system detects that the electrode pads have been correctly attached. The "AED Training" page displays "Electrode Pads Attached Correctly" The video window starts playing video segment AEDIV. The speaker begins playing the instructional audio: "The electrode pads have been correctly attached. Now, do not touch the manikin. ECG analysis in progress" A few seconds later, the speaker begins playing the instructional audio: "Shock advised. Please press the shock button to administer the shock".
Step 5: The user presses the shock button on the AED simulation machine. The system detects that the shock button has been correctly pressed. The "AED Training" page displays "Shock Button Pressed Correctly" The video window starts playing video segment AED V. The speaker begins playing the instructional audio: "The shock button has been correctly pressed. Shock completed. Now, please properly dispose of the electrode pads, return them to the AED simulation machine, and close the cover of the AED simulation machine".
Step 6: After the user closes the cover of the AED simulation machine, the video window starts playing video segment AED6. The speaker begins playing the instructional audio: "AED training ended. Now, please click 'Exit Training'" .
Step 7: After the user clicks "Exit Training," the video starts playing the "Close Cabinet Door" video segment. The speaker begins playing the instructional audio: "Please slowly lift the cabinet door and close it firmly" When the "Close Cabinet Door" video segment ends, the system returns to the main page.

Further Improvement to the Above Technical Solution: The AED assessment method includes the following steps:
Step 1: After the user completes the AED training, the user clicks on "AED Assessment" on the main page of the touch display screen. The system displays a dialog box: "Please place your ID card in the ID card scanning area". The speaker begins playing the instructional audio: "Please place your ID card in the ID card scanning area". If the ID card reader does not recognize a valid ID card within 30 seconds, the dialog box closes, and the system returns to the "Main Page".
Step 2: After the user successfully scans their ID card, the system navigates to the "AED Assessment" page. The speaker begins playing the instructional audio: "AED assessment requires you to use the AED simulation machine to perform simulated defibrillation on the simulation manikin. Are you ready? If you are ready, please click 'Start Assessment'" .
Step 3: After the user clicks "Start Assessment," the speaker begins playing the instructional audio: "The electric door lock has been opened. Please slowly pull open the cabinet door and lay it flat on the ground. Now, please kneel on the foam pad on the cabinet door and begin the operation".
Step 4: After the user opens the cover of the AED simulation machine, the "AED Assessment" page displays "Cover Opened Correctly" The speaker begins playing the instructional audio: "Now, please continue the operation".
Step 5: The user attaches the two electrode pads. Once the system detects that the electrode pads have been correctly attached, the "AED Assessment" page displays "Electrode Pads Attached Correctly". The speaker begins playing the instructional audio: "Now, do not touch the manikin. ECG analysis in progress". A few seconds later, the speaker begins playing the instructional audio: "Shock advised. Please press the shock button to administer the shock".
Step 6: The user presses the shock button on the AED simulation machine. The system detects that the shock button has been correctly pressed. The "AED Training" page displays "Shock Button Pressed Correctly" The speaker begins playing the instructional audio: "Shock completed. Now, please properly dispose of the electrode pads, return them to the AED simulation machine, and close the cover of the AED simulation machine".
Step 7: After the user closes the cover of the AED simulation machine, the speaker begins playing the instructional audio: "AED assessment ended. Now, please click 'Exit Assessment'".
Step 8: After the user clicks "Exit Assessment," the speaker begins playing the instructional audio: "Please slowly lift the cabinet door and close it firmly". The system returns to the main page.

### Benefits and Advantages

The advantages and positive effects of The present invention compared to existing technologies are:
The structure of the smart CPR training and assessment system is simple, rational, and has multiple functions. It is easy to operate, has a relatively low overall cost, is easy to maintain, and is beneficial for widespread application.

The touch display screen and control system are configured as a tablet computer. The tablet computer is hinged to the cabinet body, allowing the tilt angle of the tablet computer to be adjusted for easy viewing by the operator.

The tablet computer includes a main screen and an additional screen. The additional screen is mounted on the inner side of the flip door next to the CPR simulation manikin and is connected to the tablet computer's main unit. This makes it convenient for users to watch and listen to the system's video and audio playback.

The CPR training and assessment methods are detailed and specific, making it easy to quickly master the operation methods.

The AED training and assessment methods are detailed and specific, making it easy to quickly master the operation methods.

The AED training and assessment methods are detailed and specific, helping trainees quickly master the operation methods.

The smart CPR training and assessment system has an alarm on the top of the cabinet body. The alarm is triggered when the door of the AED storage box is opened, serving as an anti-theft and warning function.

### Brief Description of the Drawings

Fig.1 is a front view of the smart cardiopulmonary resuscitation (CPR) training and assessment system of the present invention.
Fig.2 is a perspective view of the smart CPR training and assessment system with the cabinet door open.
Fig.3 is a perspective view of the AED simulation machine in the smart CPR training and assessment system.
Fig.4 is a schematic diagram of the interface positions of the CPR simulation manikin in the smart CPR training and assessment system.
Fig.5 is an exploded view of the CPR simulation manikin in the smart CPR training and assessment system.
Fig.6 is a structural principle diagram of the smart CPR training and assessment system.

In which: 1-Touch Display Screen; 1.1-External Door Handle; 1.2-Internal Door Handle; 2-Human Body Sensor; 3-Alarm; 4-Camera; 5-Antenna; 6-ID Card Reader; 7-Cabinet Body; 7.1-Cabinet Handle; 8-AED Storage Box; 8.1-Electric Lock; 9-Downward-Opening Door; 9.1-External Handle; 9.2-Internal Handle; 10-Speaker; 11-CPR Simulation Manikin; 11.1-Second Aviation Socket; 11.2-Indicator Light; 11.3-Compression Spring; 11.4-Chest Board; 11.5-Protective Cover; 11.6-AED Electrode Pad Position Detection Device; 11.7-Compression Depth Sensor; 11.8-Illuminated Button Switch; 11.9-First Aviation Socket; 11.10-Compression Board; 11.11-CPR Simulation Manikin Control Circuit Board; 12-Disinfectant Bottle; 13-Disposable CPR Barrier Disinfectant Mask Box; 14-AED Simulation Machine; 14.1-Button Switch; 14.2-Electrode Pad; 15-Foam Pad; 16-Hinge; 17-Hydraulic Rod; 18-Signal Line; 18.1-Nylon Waterproof Connector; 18.2-Second Aviation Plug.

### Detailed Description

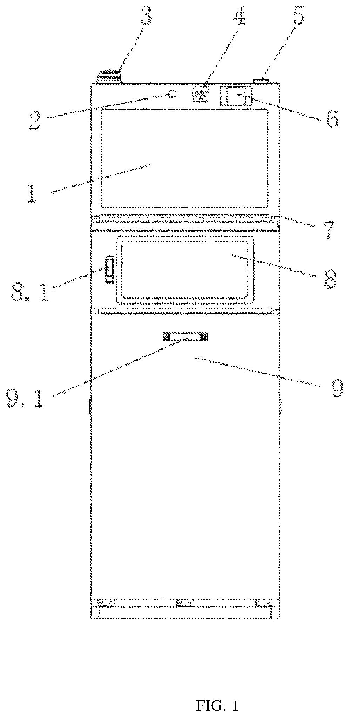

The following is a further detailed description of the present invention with reference to the drawings:
Referring to Fig. 1-6, an embodiment of the smart cardiopulmonary resuscitation (CPR) training and assessment system of the present invention includes a cabinet body 7, a CPR simulation manikin 11, an AED simulation machine 14, a switch power supply, a touch display screen 1, and a control system. The upper part of the cabinet body 7 has a front panel equipped with a human body sensor 2, a camera 4, and an ID card reader 6. The upper part of the cabinet body 7 has two side walls, each equipped with a speaker 10. The AED simulation machine 14, the touch display screen 1, the speakers 10, the human body sensor 2, the camera 4, and the ID card reader 6 are all connected to the control system. The touch display screen 1 and the control system are located in the upper part of the cabinet body 7. A cabinet door in front of a compartment of a lower part of the cabinet body 7 opens downward. The CPR simulation manikin 11 and the AED simulation machine 14 are mounted on the inner side of the downward-opening door 9. The CPR simulation manikin 11 contains a CPR simulation manikin control circuit board 11.11, which is connected to the control system via a serial communication cable. The control system includes a CPR training module, a CPR assessment module, an AED training module, an AED assessment module, a touch screen driver module, and a video playback module.

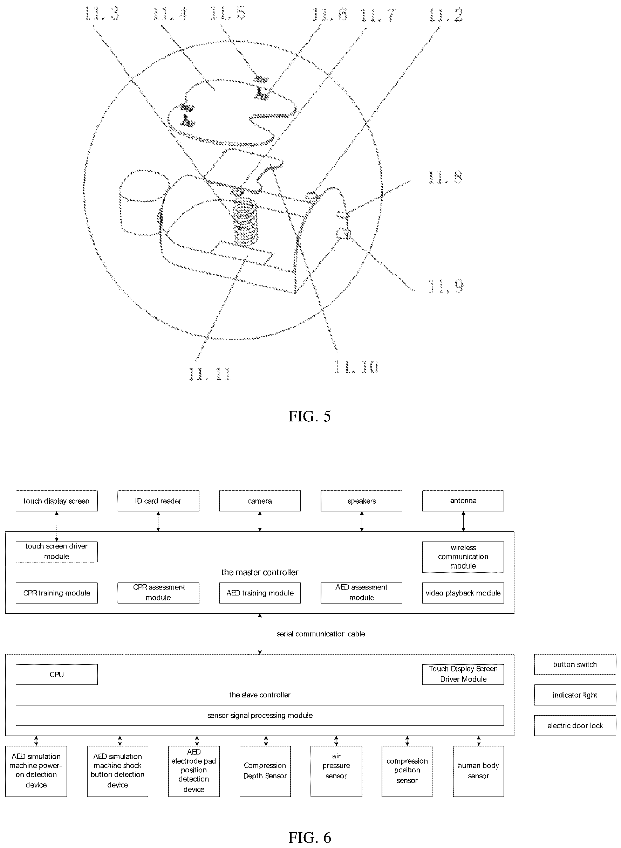

Furthermore, as shown in Figures 1 and 5, the CPR simulation manikin 11 includes a head shell and a torso shell. The torso shell contains a compression spring 11.3, with the lower end fixed to the inner back side of the torso shell. The upper end of the compression spring 11.3 is sequentially fitted with a compression board 11.10 and a chest board 11.4. Below the compression board 11.10, there is a compression depth sensor 11.7, which detects the depth of hand pressure applied by the user during CPR training or assessment. The bottom surface of the chest board 11.4 has a compression position sensor, which detects the position of the user's hand pressure on the chest board 11.4 during CPR training or assessment. The head shell has a breathing tube inserted into its mouth. The CPR simulation manikin control circuit board 11.11 has an air pressure sensor, with the breathing tube connected to the air pressure sensor's air intake port to measure the blowing pressure during artificial respiration. The CPR simulation manikin control circuit board 11.11 includes a central controller and a sensor signal processing module which is connected to the compression depth sensor 11.7, the compression position sensor, and the air pressure sensor. The torso shell of the CPR simulation manikin 11 has an indicator light 11.2 and an illuminated button switch 11.8. Pressing the illuminated button switch 11.8 connects the CPR simulation manikin 11 to the switch power supply, and the indicator light 11.2 lights up.

Specifically, as shown in Fig. 5, the CPR simulation manikin control circuit board 11.11 is mounted on the inner back side of the torso shell. The compression depth sensor 11.7 uses an ultrasonic probe, which includes an ultrasonic transmitter end mounted on the bottom surface of the compression board 11.10 and an ultrasonic receiver end mounted on the CPR simulation manikin control circuit board 11.11. The compression position sensor includes a circuit board and several conductive silicone rubber blocks housed in an open-ended shell. The conductive silicone rubber blocks are embedded in the middle and peripheral parts of the bottom surface of the chest board 11.4. The open-ended shell is detachably mounted on the bottom surface of the chest board 11.4, covering all the conductive silicone rubber blocks. The circuit board is connected to the sensor signal processing module. The compression board 11.10 is made of plastic, and the chest board 11.4 is made of silicone, with the chest board 11.4 covering the compression board 11.10. The chest board 11.4 is covered with a chest skin.

During CPR training or assessment, the operator presses the chest board 11.4 and the compression board 11.10 with their hands. The ultrasonic transmitter end on the compression board 11.10 emits ultrasonic waves, which are received by the ultrasonic receiver end on the CPR simulation manikin control circuit board 11.11. The CPR simulation manikin control circuit board 11.11 calculates the distance between the compression board 11.10 and the CPR simulation manikin control circuit board 11.11 based on the time from transmission to reception and sends this data to the control system. The control system determines whether the compression depth is correct, too deep, or too shallow based on the set distance values and provides audio feedback through the speakers 10. When the operator presses the chest board 11.4, if the pressure is applied to the middle of the chest board 11.4, the conductive silicone rubber blocks in the middle of the bottom surface of the chest board 11.4 contact the middle of the circuit board inside the open-ended shell, triggering the adjacent circuit to conduct. The CPR simulation manikin control circuit board 11.11 receives the signal that the middle circuit is conducting, indicating that the compression position is correct. If the pressure is applied to the periphery of the chest board 11.4, the conductive silicone rubber blocks on the periphery of the bottom surface of the chest board 11.4 contact the periphery of the circuit board inside the open-ended shell, triggering the adjacent circuit to conduct. The CPR simulation manikin control circuit board 11.11 receives the signal that the peripheral circuit is conducting, indicating that the compression position is off-center, and the control system provides audio feedback through the speakers 10.

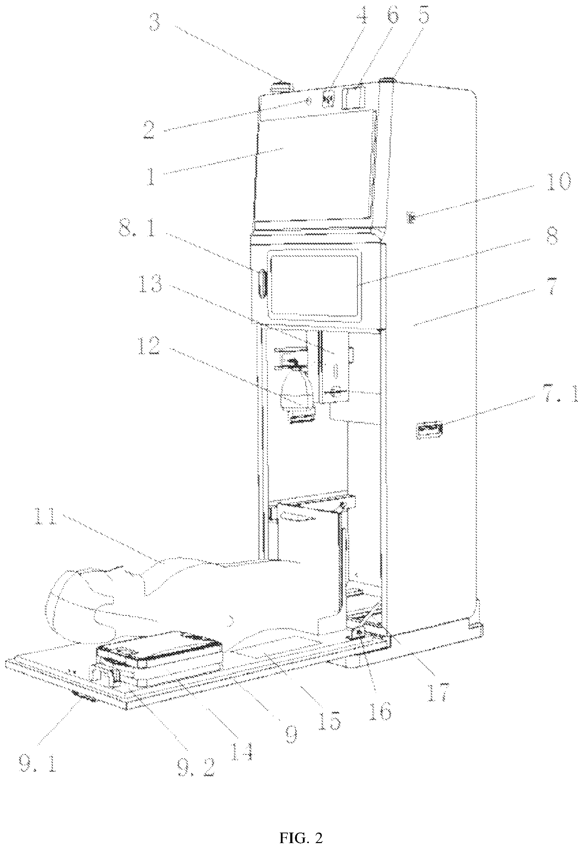

As shown in Fig.2, 3, and 5, the AED simulation machine 14 includes a box, a main unit inside the box, and two electrode pads 14.2 connected to the main unit. The two electrode pads 14.2 are used to attach to the chest board 11.4 of the CPR simulation manikin 11 for simulated defibrillation. The box has a cover, and the main unit has a button switch 14.1 and a shock button. The box is mounted on the inner side of the downward-opening door 9 near the head shell of the CPR simulation manikin 11. The chest board 11.4 has two AED electrode pad position sensors 11.6 to detect the position signals of the two electrode pads 14.2 attached to the AED simulation machine 14. The CPR simulation manikin control circuit board 11.11, which is mounted on the inner back side of the torso shell, includes a central controller and a sensor signal processing module that is connected to the AED electrode pad position sensors 11.6. A protective cover can be placed over the AED electrode pad position detection device 11.6.

Preferably, the AED electrode pad position sensors 11.6 include two Hall sensors mounted on the chest board 11.4 and magnets mounted on the two electrode pads 14.2.

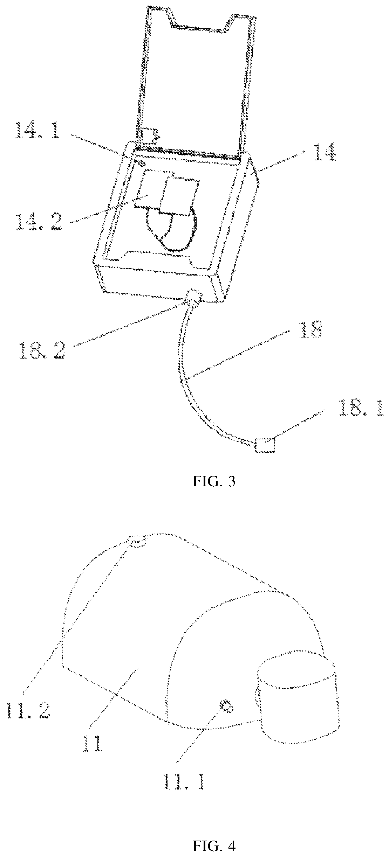

As shown in Fig. 4, the torso shell of the CPR simulation manikin 11 has a first aviation socket 11.9 and a second aviation socket 11.1, both connected to the sensor signal processing module of the CPR simulation manikin control circuit board 11.11. During use, the first aviation socket 11.9 on the CPR simulation manikin 11 is plugged into the first aviation plug from the control system. The signal line 18 from the AED simulation machine 14's main unit has a second aviation plug 18.2 at one end, which is plugged into the second aviation socket 11.1 on the CPR simulation manikin 11. The second aviation plug 18.2 has a nylon waterproof connector 18 at the end connected to the AED simulation machine 14's main unit..

Furthermore, as shown in Fig. 1, the cabinet body 7 is a vertical rectangular shell. The middle part of the cabinet body 7 includes an AED storage box 8 with a door at the front. The AED storage box 8 houses an AED. The switch power supply is located in the upper compartment of the cabinet body 7 and is used to connect to the mains power supply, converting AC to low-voltage DC to power the smart CPR training and assessment system. The top of the cabinet body 7 has an alarm 3 connected to the switch power supply via a power cord. The door of the AED storage box 8 has an electric lock 8.1 connected to the control system, allowing the control system to control the electric lock 8.1 to open the door and activate the alarm 3.

Specifically, as shown in Fig. 2, each side edge of the inner side of the downward-opening door 9 has a hinge 16. Two hydraulic rods 17 are connected to the two hinges 16 at one end and hinged to the corresponding inner side of the lower cabinet compartment at the other end. The hydraulic rods 17 use hydraulic dampers to slow down the opening speed of the downward-opening door 9, ensuring a smooth opening and gentle landing process. The downward-opening door 9 or the cabinet body 7 has an electric door lock connected to the control system via the CPR simulation manikin control circuit board, allowing the control system to control the electric door lock to automatically open the downward-opening door 9.

To facilitate the opening and closing of the downward-opening door 9, the outer and inner sides of one end of the downward-opening door 9 have external handles 9.1 and internal handles 9.2. As shown in Fig. 2, to create comfortable operating conditions for the user, a foam pad 15 is placed on the inner side of the downward-opening door 9 near the torso shell of the CPR simulation manikin 11. Preferably, the foam pad 15 has a thickness of 10-20 millimeters, a length of 400-600 millimeters, and a width of 200-400 millimeters. During use, the user kneels on the foam pad 15, reducing the risk of knee injury. The sides of the cabinet body 7 have cabinet handles 7.1 for easy movement of the cabinet.

The touch display screen 1 and the control system are configured as a tablet computer. The top of the cabinet body 7 has an antenna 5 for the tablet computer to connect to a wireless local area network via Wi-Fi.

Preferably, the tablet computer includes a main screen and an additional screen. The main screen and the tablet computer's main unit are integrated and located in the upper compartment of the cabinet body 7. The tablet computer can also be hinged to the cabinet body 7 to adjust the tilt angle. The additional screen is mounted on the inner side of the downward-opening door 9 and is detachably connected to the downward-opening door 9. The additional screen is connected to the tablet computer's main unit.

As shown in Fig. 2, the inner side wall of the lower compartment of the cabinet body 7 has a disinfectant bottle 12 and a disposable CPR barrier disinfectant mask box 13 to ensure standard operation during training and assessment, avoid secondary contamination, and ensure user safety.

As shown in Fig. 6, the control system acts as the master controller, and the CPR simulation manikin control circuit board 11.11 acts as the slave controller, connected via a serial communication cable. The control system also includes a wireless communication module connected to the antenna 5. The CPR simulation manikin control circuit board 11.11 is also connected to the button switch 14.1, the indicator light 11.2, and the electric door lock. The sensor signal processing module of the CPR simulation manikin control circuit board 11.11 is also connected to the AED electrode pad position detection device 11.6, the AED simulation machine shock button detection device, and the AED simulation machine power-on detection device. The AED simulation machine shock button detection device detects whether the shock button on the AED simulation machine is pressed and sends the signal to the control system. The AED simulation machine power-on detection device detects whether the button switch 14.1 on the AED simulation machine is activated and sends the signal to the control system.

Referring to Fig.1 to Fig.6, an implementation example of the CPR training and assessment method for the smart CPR training and assessment system includes a CPR training method and a CPR assessment method. The CPR training method includes the following steps:
Step 1: After powering on, the touch display screen displays the main page. The user taps "CPR Training" on the main page, and the system navigates to the "CPR Training" page. The video window on the touch display screen starts playing video segment CPR I . The speaker 10 begins playing the instructional audio: "CPR training requires you to perform chest compressions and artificial respiration on the simulation manikin. Are you ready? If you are ready, please tap 'Start Training'"
Step 2: After the user taps "Start Training," the video window starts playing video segment CPR II. The speaker 10 begins playing the instructional audio: "The electric door lock has been opened. Please slowly pull open the cabinet door and lay it flat on the ground. Now, please kneel on the foam pad on the cabinet door with both knees, place your hands crossed on the midpoint of the line connecting the two nipples on the manikin's chest, and begin 30 chest compressions. The compression depth should be 5 to 6 centimeters. Please follow the rhythm of the sound" This audio is played at least 2 times.
Step 3: The user begins the chest compression action on the CPR simulation manikin. Once the system detects that the user has started the chest compression action, the video window starts playing video segment CPRIII, which demonstrates the correct chest compression technique. The speaker 10 begins playing a rhythmic sound at a frequency of 110 beats per minute. The system monitors the user's compression action and provides real-time audio feedback through the speaker 10 to correct the user's action: "Compression too deep," "Compression too shallow," "Insufficient recoil," or "Incorrect compression position"
Step 4: After the user completes 30 correct chest compressions, the video window starts playing video segment CPRIV. The speaker 10 begins playing the instructional audio: "30 chest compressions completed. Please take a disinfectant mask from the disinfectant mask box, open the small package, place the mask on the CPR simulation manikin's mouth, and perform two artificial respirations"
Step 5: The user begins the artificial respiration action on the CPR simulation manikin. Once the system detects that the user has started the artificial respiration action, the video window starts playing video segment CPR V, which demonstrates the correct artificial respiration technique. The system monitors the user's artificial respiration action in real-time and provides real-time audio reminders through the speaker 10: "Correct breathing," "Breathing too strong," or "Breathing too weak"
Step 6: After the user completes 2 correct artificial respirations, the video window starts playing video segment CPRIII, which demonstrates the correct chest compression technique. The speaker 10 begins playing the instructional audio: "Two artificial respirations completed. Please continue with 30 chest compressions"
Step 7: Repeat steps 3, 4, 5, and 6 until the user taps "Exit Training" The video window starts playing the "Close Cabinet Door" video segment. The speaker 10 begins playing the instructional audio: "Please slowly lift the cabinet door and close it firmly" When the "Close Cabinet Door" video segment ends, the system returns to the main page..

Furthermore, the CPR assessment method includes the following steps:
Step 1: The user taps "CPR Assessment" on the main page of the touch display screen, and the system enters the identity recognition step. The system displays a dialog box: "Please place your ID card in the ID card scanning area" The speaker 10 begins playing the instructional audio: "Please place your ID card in the ID card scanning area," repeated at least twice. If the ID card reader does not recognize a valid ID card within 30 seconds, the dialog box closes, and the system returns to the "Main Page"
Step 2: After the user successfully scans their ID card, the system navigates to the "CPR Assessment" page. The speaker 10 begins playing the instructional audio: "CPR assessment requires you to perform chest compressions and artificial respiration on the simulation manikin. Are you ready? If you are ready, please tap 'Start Assessment'"
Step 3: After the user taps "Start Assessment," the speaker 10 begins playing the instructional audio: "The electric door lock has been opened. Please slowly pull open the cabinet door and lay it flat on the ground. Now, please kneel on the foam pad on the cabinet door and begin 30 chest compressions," repeated at least 2 times.
Step 4: The user begins the chest compression action. Once the system detects that the user has started the chest compression action, the speaker 10 plays a rhythmic sound at a frequency of 110 beats per minute but does not provide real-time feedback on the user's compression action.
Step 5: After the user completes 30 chest compressions, the speaker 10 begins playing the instructional audio: "30 chest compressions completed. Please take a disinfectant mask from the disinfectant mask box, open the small package, place the mask on the CPR simulation manikin's mouth, and perform two artificial respirations"
Step 6: The user begins the artificial respiration action on the CPR simulation manikin.
Step 7: After the user completes 2 artificial respirations, the speaker 10 begins playing the instructional audio: "Two artificial respirations completed. Assessment [successful/failed]. Detailed scores are shown on the screen. CPR assessment ended. Now, please tap 'Exit Assessment'"
Step 8: After the user taps "Exit Assessment," the speaker 10 begins playing the instructional audio: "Please slowly lift the cabinet door and close it firmly," repeated at least twice. The system then returns to the main page.

Referring to Fig.1-6, an implementation example of the AED training and assessment method for the smart CPR training and assessment system includes an identity recognition step, an AED training method, and an AED assessment method. First, the identity recognition step is performed, followed by the AED training method, and then the AED assessment method. The AED training method includes the following steps:
Step 1: After the identity recognition step is completed, the touch display screen 1 displays the main page. The user taps "AED Training" on the main page, and the system navigates to the "AED Training" page. The video window on the touch display screen 1 starts playing video segment AED I . The speaker 10 begins playing the instructional audio: "AED training requires you to use the AED simulation machine to perform simulated defibrillation on the CPR simulation manikin. Are you ready? If you are ready, please tap 'Start Training'"
Step 2: After the user taps "Start Training," the video window starts playing video segment AED II. The speaker 10 begins playing the instructional audio: "The electric door lock has been opened. Please slowly pull open the cabinet door and lay it flat on the ground. Now, please kneel on the foam pad on the cabinet door and gently press the cover of the AED simulation machine to open it"
Step 3: After the user opens the cover of the AED simulation machine 14, the "AED Training" page displays "Cover Opened Correctly" The video window starts playing video segment AEDIII. The speaker 10 begins playing the instructional audio: "The cover of the AED simulation machine has been correctly opened. Now, please take out the two electrode pads, peel off the adhesive film, and correctly attach them to the manikin's skin as indicated on the electrode pads"
Step 4: After the user attaches the two electrode pads, the system detects that the electrode pads have been correctly attached. The "AED Training" page displays "Electrode Pads Attached Correctly" The video window starts playing video segment AEDIV. The speaker 10 begins playing the instructional audio: "The electrode pads have been correctly attached. Now, do not touch the manikin. ECG analysis in progress" A few seconds later, the speaker 10 begins playing the instructional audio: "Shock advised. Please press the shock button to administer the shock"
Step 5: The user presses the shock button on the AED simulation machine 14. The system detects that the shock button has been correctly pressed. The "AED Training" page displays "Shock Button Pressed Correctly" The video window starts playing video segment AED V. The speaker 10 begins playing the instructional audio: "The shock button has been correctly pressed. Shock completed. Now, please properly dispose of the electrode pads, return them to the AED simulation machine, and close the cover of the AED simulation machine"
Step 6: After the user closes the cover of the AED simulation machine 14, the video window starts playing video segment AEDVI. The speaker 10 begins playing the instructional audio: "AED training ended. Now, please tap 'Exit Training'"
Step 7: After the user taps "Exit Training," the video starts playing the "Close Cabinet Door" video segment. The speaker 10 begins playing the instructional audio: "Please slowly lift the cabinet door and close it firmly" When the "Close Cabinet Door" video segment ends, the system returns to the main page.

Furthermore, the AED assessment method includes the following steps:
Step 1: After the user completes the AED training, the user clicks on "AED Assessment" on the main page of the touch display screen 1. The system displays a dialog box: "Please place your ID card in the ID card scanning area" The speaker 10 begins playing the instructional audio: "Please place your ID card in the ID card scanning area," repeated twice. If the ID card reader 6 does not recognize a valid ID card within 30 seconds, the dialog box closes, and the system returns to the "Main Page"
Step 2: After the user successfully scans their ID card, the system navigates to the "AED Assessment" page. The speaker 10 begins playing the instructional audio: "AED assessment requires you to use the AED simulation machine to perform simulated defibrillation on the simulation manikin. Are you ready? If you are ready, please click 'Start Assessment'"
Step 3: After the user clicks "Start Assessment," the speaker 10 begins playing the instructional audio: "The electric door lock has been opened. Please slowly pull open the cabinet door and lay it flat on the ground. Now, please kneel on the foam pad on the cabinet door and begin the operation," repeated at least 2 times.
Step 4: After the user opens the cover of the AED simulation machine 14, the "AED Assessment" page displays "Cover Opened Correctly" The speaker 10 begins playing the instructional audio: "Now, please continue the operation"
Step 5: The user attaches the two defibrillation electrode pads 14.2. Once the system detects that the defibrillation electrode pads 14.2 have been correctly attached, the "AED Assessment" page displays "Electrode Pads Attached Correctly" The speaker 10 begins playing the instructional audio: "Now, do not touch the manikin. ECG analysis in progress" A few seconds later, the speaker 10 begins playing the instructional audio: "Shock advised. Please press the shock button to administer the shock"
Step 6: The user presses the shock button on the AED simulation machine 14. The system detects that the shock button has been correctly pressed. The "AED Training" page displays "Shock Button Pressed Correctly" The speaker 10 begins playing the instructional audio: "Shock completed. Now, please properly dispose of the electrode pads, return them to the AED simulation machine, and close the cover of the AED simulation machine"
Step 7: After the user closes the cover of the AED simulation machine 14, the speaker 10 begins playing the instructional audio: "AED assessment ended. Now, please click 'Exit Assessment‴
Step 8: After the user clicks "Exit Assessment," the speaker 10 begins playing the instructional audio: "Please slowly lift the cabinet door and close it firmly," repeated at least twice. The system then returns to the main page.

Furthermore, during the identity recognition process, if the personal information on the ID card is normal, the device switch can be opened to start the device. Otherwise, the device switch cannot be opened to start the device.

The above description is not a limitation of the present invention. The present invention is not limited to the above examples. Modifications, changes, additions, or substitutions made by those skilled in the art within the scope of the present invention also fall within the scope of protection of the present invention.

## Claims

1. A smart cardiopulmonary resuscitation "CPR" training and assessment system, comprising:
a cabinet body, a CPR simulation manikin, an AED simulation machine, a switch power supply, a touch display screen, and a control system, wherein a front panel of an upper part of the cabinet body is equipped with a human body sensor, a camera, and an ID card reader; the upper part of the cabinet body has two side walls, each equipped with a speaker; the AED simulation machine, the touch display screen,
the speakers, the human body sensor, the camera, and the ID card reader are all connected to the control system; the touch display screen and the control system are located in the upper part of the cabinet body; a cabinet door in front of a compartment of a lower part of the cabinet body opens downward; the CPR simulation manikin and the AED simulation machine are mounted on an inner side of the downward-opening door; the CPR simulation manikin contains a CPR simulation manikin control circuit board connected to the control system via a serial communication cable; the control system includes a CPR training module, a CPR assessment module, an AED training module, an AED assessment module, a touch screen driver module, and a video playback module.

2. The smart CPR training and assessment system according to claim 1, wherein the CPR simulation manikin includes a head shell and a torso shell; the torso shell contains a compression spring, a lower end of the compression spring is fixed to an inner back side of the torso shell; an upper end of the compression spring is sequentially fitted with a compression board and a chest board; below the compression board is a compression depth sensor configured to detect a depth of hand pressure applied by a user during CPR training or assessment; a bottom surface of the chest board has a compression position sensor configured to detect a position of the user's hand pressure on the chest board during CPR training or assessment; a breathing tube is inserted into a mouth of the head shell; an air pressure sensor is arranged on the CPR simulation manikin control circuit board, the breathing tube is connected to an air intake port of the air pressure sensor to measure a blowing pressure during artificial respiration; the CPR simulation manikin control circuit board includes a central controller and a sensor signal processing module, the sensor signal processing module is connected to the compression depth sensor, the compression position sensor, and the air pressure sensor.

3. The smart CPR training and assessment system according to claim 2, wherein the CPR simulation manikin control circuit board is mounted on the inner back side of the torso shell; the compression depth sensor uses an ultrasonic probe including an ultrasonic transmitter end mounted on a bottom surface of the compression board and an ultrasonic receiver end mounted on the CPR simulation manikin control circuit board; the compression position sensor includes a circuit board and several conductive silicone rubber blocks housed in an open-ended shell; the conductive silicone rubber blocks are embedded in middle and peripheral parts of the bottom surface of the chest board; the open-ended shell is detachably mounted on the bottom surface of the chest board, covering all the conductive silicone rubber blocks; the circuit board is connected to the sensor signal processing module; the compression board is made of plastic, and the chest board is made of silicone, with the chest board covering the compression board; the chest board is covered with a chest skin.

4. The smart CPR training and assessment system according to claim 2 or 3, wherein the AED simulation machine includes a box, a main unit inside the box, and two electrode pads connected to the main unit; the box has a cover, and the main unit has a button switch and a shock button; the box is mounted on the inner side of the downward-opening door near a head of the CPR simulation manikin; the chest board has two AED electrode pad position sensors to detect position signals of the two electrode pads attached to the AED simulation machine; the CPR simulation manikin control circuit board includes a central controller and a sensor signal processing module, the sensor signa processing model is connected to the AED electrode pad position sensors.

5. The smart CPR training and assessment system according to claim 4, wherein the AED electrode pad position sensors include two Hall sensors mounted on the chest board and magnets mounted on the two electrode pads; the CPR simulation manikin control circuit board is mounted on the inner back side of the torso shell; the compression board is made of plastic, and the chest board is made of silicone, with the chest board covering the compression board; the chest board is covered with a chest skin.

6. The smart CPR training and assessment system according to claim 4, wherein the torso shell of the CPR simulation manikin has a first aviation socket and a second aviation socket, both connected to the sensor signal processing module of the CPR simulation manikin control circuit board; an end of a signal line connected to the main unit of the AED simulation machine is connected to a second aviation plug plugging into the second aviation socket ; a first aviation plug connected to the control system plugging into the first aviation socket.

7. The smart CPR training and assessment system according to any one of claims 1-3, wherein the cabinet body is a vertical rectangular shell; a middle part of the cabinet body is an AED storage box with a door at a front; the AED storage box houses an AED; a top of the cabinet body has an alarm connected to the switch power supply via a power cord; the door of the AED storage box has an electric lock connected to the control system, allowing the control system to control the electric lock to open the door and activate the alarm.

8. The smart CPR training and assessment system according to any one of claims 1-3, wherein each side edge of the inner side of the downward-opening door has a hinge; a hydraulic rod is connected to the hinge at one end and hinged to a corresponding inner side of the compartment at another end; the hydraulic rod uses a hydraulic damper; the downward-opening door or the cabinet body has an electric door lock connected to the control system via the CPR simulation manikin control circuit board, allowing the control system to control the electric door lock to automatically open the downward-opening door.

9. The smart CPR training and assessment system according to any one of claims 1-3, wherein the inner side of the downward-opening door near the torso shell of the CPR simulation manikin has a foam pad; an inner side wall of the compartment is equipped with a disinfectant bottle and a disposable CPR barrier disinfectant mask box.

10. The smart CPR training and assessment system according to any one of claims 1-3, wherein the touch display screen and the control system are configured as a tablet computer; an antenna is arranged on a top of the cabinet body to enable the tablet computer to connect to a wireless local area network via WiFi; the control system also includes a wireless communication module connected to the antenna.

11. The smart CPR training and assessment system according to any one of claims 1-3, wherein the tablet computer includes a main screen and an additional screen; the main screen and the tablet computer's main unit are integrated and located in a compartment in the upper part of the cabinet body; the additional screen is mounted on the inner side of the downward-opening door and is detachably connected to the downward-opening door; the additional screen is connected to the tablet computer's main unit.

12. A training and assessment method for the smart CPR training and assessment system according to any one of claims 1-11, wherein the training and assessment method includes a CPR training method and a CPR assessment method; the CPR training method includes the following steps:
Step 1: After powering on, the touch display screen shows a main page; the user clicks on "CPR Training" on the main page, and the system navigates to the "CPR Training" page; a video window on the touch display screen starts playing video segment CPR I ; the speaker begins playing instructional audio: "CPR training requires you to perform chest compressions and artificial respiration on the simulation manikin; are you ready? If you are ready, please click 'Start Training'";
Step 2: After the user clicks "Start Training," the video window starts playing video segment CPR II ; the speaker begins playing the instructional audio: "The electric door lock has been opened. Please slowly pull open the cabinet door and lay it flat on the ground. Now, please kneel on the foam pad on the cabinet door with both knees, place your hands crossed on the midpoint of the line connecting the two nipples on the manikin's chest, and begin 30 chest compressions; the compression depth should be 5 to 6 centimeters. Please follow the rhythm of the sound", this audio is played at least 2 times;
Step 3: The user begins the chest compression action on the CPR simulation manikin; once the system detects that the user has started the chest compression action, the video window starts playing video segment CPRIII, which demonstrates the correct chest compression technique; the speaker begins playing a rhythmic sound at a frequency of 100-120 beats per minute; the system monitors the user's compression action and provides real-time audio feedback through the speaker to correct the user's action: "Compression too deep," "Compression too shallow," "Insufficient recoil," or "Incorrect compression position";
Step 4: After the user completes 30 correct chest compressions, the video window starts playing video segment CPRIV; the speaker begins playing the instructional audio: "30 chest compressions completed. Please take a disinfectant mask from the disinfectant mask box, open the small package, place the mask on the CPR simulation manikin's mouth, and perform two artificial respirations";
Step 5: The user begins the artificial respiration action on the CPR simulation manikin; once the system detects that the user has started the artificial respiration action, the video window starts playing video segment CPRV, which demonstrates the correct artificial respiration technique; the system monitors the user's artificial respiration action in real-time and provides real-time audio reminders through the speaker: "Correct breathing," "Breathing too strong," or "Breathing too weak";
Step 6: After the user completes 2 correct artificial respirations, the video window starts playing video segment CPRIII, which demonstrates the correct chest compression technique; the speaker begins playing the instructional audio: "Two artificial respirations completed. Please continue with 30 chest compressions";
Step 7: Repeat steps 3, 4, 5, and 6 until the user clicks "Exit Training"; the video window starts playing the "Close Cabinet Door" video segment; the speaker begins playing the instructional audio: "Please slowly lift the cabinet door and close it firmly"; when the "Close Cabinet Door" video segment ends, the system returns to the main page.

13. The training and assessment method for the smart CPR training and assessment system according to claim 12, wherein the CPR assessment method includes the following steps:
Step 1: The user clicks on "CPR Assessment" on the main page of the touch display screen, and the system enters the identity recognition step; the system displays a dialog box: "Please place your ID card in the ID card scanning area"; the speaker begins playing the instructional audio: "Please place your ID card in the ID card scanning area"; if the ID card reader does not recognize a valid ID card within 30 seconds, the dialog box closes, and the system returns to the "Main Page";
Step 2: After the user successfully scans their ID card, the system navigates to the "CPR Assessment" page; the speaker begins playing the instructional audio: "CPR assessment requires you to perform chest compressions and artificial respiration on the simulation manikin; are you ready? If you are ready, please click 'Start Assessment'" ;
Step 3: After the user clicks "Start Assessment", the speaker begins playing the instructional audio: "The electric door lock has been opened. Please slowly pull open the cabinet door and lay it flat on the ground. Now, please kneel on the foam pad on the cabinet door and begin 30 chest compressions";
Step 4: The user begins the chest compression action; once the system detects that the user has started the chest compression action, the speaker plays a rhythmic sound at a frequency of 100-120 beats per minute;
Step 5: After the user completes 30 chest compressions, the speaker begins playing the instructional audio: "30 chest compressions completed. Please take a disinfectant mask from the disinfectant mask box, open the small package, place the mask on the CPR simulation manikin's mouth, and perform two artificial respirations";
Step 6: The user begins the artificial respiration action on the CPR simulation manikin;
Step 7: After the user completes 2 artificial respirations, the speaker begins playing the instructional audio: "Two artificial respirations completed; assessment successful. Detailed scores are shown on the screen. CPR assessment ended. Now, please click 'Exit Assessment'" or "Two artificial respirations completed; assessment failed. Detailed scores are shown on the screen. CPR assessment ended. Now, please click 'Exit Assessment'" ;
Step 8: After the user clicks "Exit Assessment," the speaker begins playing the instructional audio: "Please slowly lift the cabinet door and close it firmly"; the system then returns to the main page.

14. The training and assessment method for the smart CPR training and assessment system according to claim 12 or 13, wherein the AED training and assessment method includes an AED training method and an AED assessment method; the AED training method includes the following steps:
Step 1: After powering on, the touch display screen shows the main page; the user clicks on "AED Training" on the main page, and the system navigates to the "AED Training" page; the video window on the touch display screen starts playing video segment AED I ; the speaker begins playing the instructional audio: "AED training requires you to use the AED simulation machine to perform simulated defibrillation on the CPR simulation manikin; are you ready? If you are ready, please click 'Start Training'";
Step 2: After the user clicks "Start Training," the video window starts playing video segment AED II; the speaker begins playing the instructional audio: "The electric door lock has been opened. Please slowly pull open the cabinet door and lay it flat on the ground. Now, please kneel on the foam pad on the cabinet door and gently press the cover of the AED simulation machine to open it";
Step 3: After the user opens the cover of the AED simulation machine, the "AED Training" page displays "Cover Opened Correctly", and the video window starts playing video segment AEDIII; the speaker begins playing the instructional audio: "The cover of the AED simulation machine has been correctly opened. Now, please take out the two electrode pads, peel off the adhesive film, and correctly attach them to the manikin's skin as indicated on the electrode pads";
Step 4: After the user attaches the two electrode pads, the system detects that the electrode pads have been correctly attached; the "AED Training" page displays "Electrode Pads Attached Correctly"; the video window starts playing video segment AEDIV; the speaker begins playing the instructional audio: "The electrode pads have been correctly attached. Now, do not touch the manikin. ECG analysis in progress"; a few seconds later, the speaker begins playing the instructional audio: "Shock advised. Please press the shock button to administer the shock";
Step 5: The user presses the shock button on the AED simulation machine; the system detects that the shock button has been correctly pressed; the "AED Training" page displays "Shock Button Pressed Correctly"; the video window starts playing video segment AEDV; the speaker begins playing the instructional audio: "The shock button has been correctly pressed. Shock completed. Now, please properly dispose of the electrode pads, return them to the AED simulation machine, and close the cover of the AED \simulation machine";
Step 6: After the user closes the cover of the AED simulation machine, the video window starts playing video segment AED6; the speaker begins playing the instructional audio: "AED training ended. Now, please click 'Exit Training'";
Step 7: After the user clicks "Exit Training," the video starts playing the "Close Cabinet Door" video segment; the speaker begins playing the instructional audio: "Please slowly lift the cabinet door and close it firmly"; when the "Close Cabinet Door" video segment ends, the system returns to the main page.

15. The training and assessment method for the smart CPR training and assessment system according to claim 14, wherein the AED assessment method includes the following steps:
Step 1: After the user completes the AED training, the user clicks on "AED Assessment" on the main page of the touch display screen; the system displays a dialog box: "Please place your ID card in the ID card scanning area"; the speaker begins playing the instructional audio: "Please place your ID card in the ID card scanning area"; if the ID card reader does not recognize a valid ID card within 30 seconds, the dialog box closes, and the system returns to the "Main Page";
Step 2: After the user successfully scans their ID card, the system navigates to the "AED Assessment" page; the speaker begins playing the instructional audio: "AED assessment requires you to use the AED simulation machine to perform simulated defibrillation on the simulation manikin; are you ready? If you are ready, please click 'Start Assessment"';
Step 3: After the user clicks "Start Assessment," the speaker begins playing the instructional audio: "The electric door lock has been opened. Please slowly pull open the cabinet door and lay it flat on the ground. Now, please kneel on the foam pad on the cabinet door and begin the operation";
Step 4: After the user opens the cover of the AED simulation machine, the "AED Assessment" page displays "Cover Opened Correctly"; the speaker begins playing the instructional audio: "Now, please continue the operation";
Step 5: The user attaches the two electrode pads; once the system detects that the electrode pads have been correctly attached, the "AED Assessment" page displays "Electrode Pads Attached Correctly"; the speaker begins playing the instructional audio: "Now, do not touch the manikin. ECG analysis in progress"; a few seconds later, the speaker begins playing the instructional audio: "Shock advised. Please press the shock button to administer the shock";
Step 6: The user presses the shock button on the AED simulation machine; the system detects that the shock button has been correctly pressed; the "AED Training" page displays "Shock Button Pressed Correctly"; the speaker begins playing the instructional audio: "Shock completed. Now, please properly dispose of the electrode pads, return them to the AED simulation machine, and close the cover of the AED simulation machine";
Step 7: After the user closes the cover of the AED simulation machine, the speaker begins playing the instructional audio: "AED assessment ended. Now, please click 'Exit Assessment'";
Step 8: After the user clicks "Exit Assessment," the speaker begins playing the instructional audio: "Please slowly lift the cabinet door and close it firmly", and the system then returns to the main page.
